# EUROPEAN PATENT SPECIFICATION

(11) **EP 3 830 068 B1**
(45) Date of publication and mention of the grant of the patent: **15.02.2023**
(21) Application number: 19745646.0
(22) Date of filing: 02.08.2019
(51) Int. Cl.: C07C 51/16, C07C 53/126, C11C 1/02, C11C 3/00

(54) **PROCESS FOR THE CLEAVAGE AND OXIDATION OF ESTERS**
VERFAHREN ZUR SPALTUNG UND OXIDIERUNG VON ESTERN
PROCÉDÉ DE CLIVAGE ET D'OXYDATION D'ESTERS

(30) Priority: 03.08.2018 EP 18187234
(43) Date of publication of application: 09.06.2021
(73) Proprietor: Völpker Spezialprodukte GmbH, 39393 Völpke (DE); Kahl GmbH & Co. KG, 22946 Trittau (DE)
(72) Inventor: HOFMANN, Tommy, 56379 Horhausen (DE); KÜHN, Fritz Elmar, 85748 Garching (DE); NAGORNY, Nicolai, 22949 Ammersbek (DE); SCHLÜTER, Marc, 22339 Hamburg (DE)
(74) Representative: Sandvoß, Stefanie
(86) International application number: PCT/EP2019/070939
(87) International publication number: WO 2020/025814

(56) References cited:
- US-A1- 2015 284 661

## Description

Waxes are used as additives or active substances in a variety of products. Large amounts of raw waxes, such as montan wax, rice bran wax, carnauba wax, or bees' wax, are refined prior to their application. The refining process may involve the cleavage of wax esters and the oxidation of the resulting alcohols to carboxylic acids in order to increase the acid number of the wax. A higher acid number, i.e. a higher concentration of acids in a wax leads to a processed wax with modified properties, e.g. in terms of the processability thereof. Thus, the cleavage and oxidation of wax esters can expand the field of use of the raw wax.

Today, a major portion of refined wax is provided using large amounts of chromosulfuric acid, i.e. a mixture of Cr(VI) reagents and sulphuric acid (e.g. DE 102 31 886 A1, DE 10 2013 007 638 A1). It can be estimated that about 60 % of the annual production of 15,000 to 25,000 tons of montan wax are refined using chromosulfuric acid, so that significant amounts of chromium and sulfuric acid are spent for this procedure. Even though methods for the recycling of chromium are meanwhile available, a reduction of the use of this heavy metal, which is classified as a substance hazardous to water, is desirable.

Moreover, the reaction of waxes with chromosulfuric acid has a limited efficiency in order to increase the acid number of the wax. Thus, many raw waxes need to be reacted with sulfuric acid (50%) and Cr(VI) reagents (such as CrO₃, Na₂Cr₂O₇) several times (cf. DE 102 31 886 A1). For each reaction, 3-6 kg of 50% sulfuric acid and 1-1.5 kg CrO₃ (or corresponding amounts of other Cr(VI) reagents) are typically used per kg of raw wax. Thus, for 1 kg of wax which is subjected to the reaction with chromosulfuric acid three times, more than 9 kg of 50% sulfuric acid and more than 3 kg CrO₃ may be required.

In order to provide rice bran wax with high acid numbers, WO 2014/060082 discloses a process wherein the wax is oxidized with chromosulfuric acid in the presence of oxidation promoters. WO 2014/060081 discloses a process wherein rice bran wax is saponified in a first step, followed by an oxidation using chromosulfuric acid. It is recommended that the saponification is carried out under pressure, since according to this application a "pressureless saponification is possible only with a considerable excess of KOH or NaOH and [...] the use of additional solvent". An alternative approach for the oxidation of wax esters not relying on the use of chromosulfuric acid is disclosed in DE 3726514, which relates to a process for the production of montan acids via oxidation of raw montan wax in the melt with water-free alkali hydroxide at elevated temperatures of app. 300 °C.

In view of the above, the present invention provides a process for the oxidation of esters, preferably of esters contained in a raw wax, which can be carried out efficiently with high yields, at moderate temperatures, without the need for high pressure, and with reduced consumption of potentially hazardous reactants.

The present invention provides a process for the oxidation of an ester via an ester cleavage reaction followed by the oxidation of the alcohol component of the ester, said process comprising
a step of subjecting a starting material comprising one or more esters of a carboxylic acid having a carbon number of 14 or more with an alcohol having a carbon number of 14 or more to an ester cleavage reaction wherein at least a part of the one or more esters is hydrolyzed to obtain a mixture of one or more carboxylic acid salts with a carbon number of 14 or more and one or more alcohols with a carbon number of 14 or more, and wherein the ester cleavage reaction is carried out under atmospheric pressure and in a temperature range of 90 to 140 °C using an aqueous base with a concentration of the base in water of 20 wt% or more;
a step of acidifying the one or more carboxylic acid salts with a carbon number of 14 or more to obtain one or more carboxylic acids; and
a step of oxidizing at least a part of the one or more alcohols with a carbon number of 14 or more to obtain one or more carboxylic acids;
wherein the step of acidifying the carboxylic acid salts and the step of oxidizing the alcohols can be carried out concurrently or successively.

The starting material which is subjected to an ester cleavage reaction in the process in accordance with the invention comprises one or more esters of a carboxylic acid having a carbon number of 14 or more with an alcohol having a carbon number of 14 or more. It will be understood in this regard that the carbon number of the carboxylic acid designates the total number of carbon atoms contained in the carboxylic acid forming the ester (also referred to herein as the carboxylic acid component of the ester), and the carbon number of the alcohol designates the total number of carbon atoms contained in the alcohol forming the ester (also referred to herein as the alcohol component of the ester).

Preferably, the starting material comprises one or more esters of a carboxylic acid with a carbon number of 16 or more and an alcohol with a carbon number of 16 or more, more preferably one or more esters of a carboxylic acid with a carbon number of 16 or more and 42 or less with an alcohol with a carbon number of 16 or more and 42 or less, and even more preferably one or more esters of a carboxylic acid with a carbon number of 20 or more and 40 or less and an alcohol of 20 or more and 40 or less.

It will be appreciated in this regard that, in the ester molecules contained in the starting material, the number of carbon atoms in the carboxylic acid component does not have to be the same as the number of carbon atoms in the alcohol component. Moreover, as indicated by the expression "one or more", the starting material may comprise mixtures of esters with different carboxylic acid components and/or different alcohol components.

In terms of the structure of the carboxylic acid and the alcohol forming the esters contained in the starting material, it is preferred that the carboxylic acids are aliphatic carboxylic acids, more preferably branched or linear, and most preferably linear carboxylic acids. While the carboxylic acids may have other functional groups besides the carboxylic acid group -COOH which is involved in the formation of the ester linkage -C(O)O- between the carboxylic acid and the alcohol, it is preferred that they have one carboxylic acid group (i.e. the carboxylic acid group which is part of the ester linkage in the ester), and no further functional groups.

With regard to the alcohol, it is preferred that the alcohols are aliphatic alcohols, more preferably branched or linear, and most preferably linear alcohols. While the alcohols may have other functional groups besides an alcoholic -OH group which is involved in the formation of the ester linkage -O(O)C- between the alcohol and the carboxylic acid, it is preferred that they have one alcoholic -OH group (i.e. the alcoholic group which is part of the ester linkage in the ester), and no further functional groups. The alcohol is preferably a primary alcohol.

In view of the above, it will be understood that a preferred starting material to be subjected to an ester cleavage reaction in the process in accordance with the invention is one which comprises one or more esters of the formula

CH₃-(CH₂)ₘ-C(O)O-(CH₂)ₙ-CH₃

wherein m+2 corresponds to the carbon number of the carboxylic acid component of the ester, and is 14 or more (i.e. m is 12 or more), and n+1 corresponds to the carbon number of the alcohol component the ester, and is also 14 or more (i.e. n is 13 or more).

Also in line with the above, m+2 is preferably 16 or more (i.e. m is 14 or more), more preferably m+2 is 16 or more and 42 or less (i.e. m is 14 or more and 40 or less), and even more preferably m+2 is 20 or more and 40 or less (i.e. m is 18 or more and 38 or less). n+1 is preferably 16 or more (i.e. n is 15 or more), more preferably n+1 is 16 or more and 42 or less (i.e. n is 15 or more and 41 or less), and even more preferably n+1 is 20 or more and 40 or less (i.e. n is 19 or more and 39 or less).

A starting material to which the process of the present invention can be applied in a particularly advantageous manner, and which comprises esters as discussed above, is a wax. Thus, it is particularly preferred that the starting material comprising one or more esters of a carboxylic acid having a carbon number of 14 or more with an alcohol having a carbon number of 14 or more, or the preferred esters discussed above, is a wax. It is even further preferred that the wax is a natural wax, such as a vegetable wax or an animal wax.

Thus, the process of the present invention is preferably a process for the oxidation of an ester which is contained in a wax (also referred to as a wax ester) via an ester cleavage reaction, followed by the oxidation of the alcohol component of the ester, to provide an oxidation product of the wax.

Examples of such a wax which are particularly suitable for use as an ester containing starting material in the process in accordance with the invention are rice bran wax, sunflower wax, carnauba wax, candelilla wax, sugarcane wax and beeswax. Among waxes, and thus among starting materials in general, rice bran wax is particularly preferred.

In addition to the esters discussed above, the starting material used in the process in accordance with the invention may comprise other compound, e.g. compounds which are typically present in natural waxes, such as non-esterified carboxylic acids or alcohols.

In the process in accordance with the invention, the starting material comprises the esters of a carboxylic acid with a carbon number of 14 or more and an alcohol with a carbon number of 14 or more in an amount of preferably 30 wt% or more, more preferably 60 wt% or more, and most preferably 80 wt% or more, based on the total weight of starting material as 100 wt%.

The ester cleavage reaction to which the starting material comprising the esters is subjected is carried out at a temperature of 80°C or more. Typically, the temperature is sufficiently high so that the starting material forms a liquid phase at the reaction temperature, i.e. that the esters to be subjected to the ester cleavage reaction are in a molten state.

According to the invention, the reaction temperature of the ester cleavage reaction is between 90°C and 140 °C. The upper limit of the reaction temperature may be adjusted to the boiling point of the aqueous base used as a reactant in the ester cleavage reaction. Preferably, the reaction is carried out at a temperature of 120°C or less. Thus, a preferred temperature range for the reaction temperature at which the ester cleavage reaction is carried out is 90 to 140°C, more preferably 100 to 140°C, still more preferably 100 to 120°C, and most preferably 110 to 120°C.

The ester cleavage reaction in accordance with the process is carried out with a concentrated aqueous base, such that the heat of reaction may lead to an increase in temperature once the reaction has started. Thus, it may be advantageous for a control of the reaction to start it at temperatures at the lower end of the ranges and the preferred ranges discussed above, and increase the temperature only slowly towards temperatures at the higher end of these ranges/preferred ranges.

In the process in accordance with the present invention, the ester cleavage reaction is carried out using an aqueous base, i.e. a base contained in water as a solvent, to which the one or more esters comprised by the starting material are exposed. During the ester cleavage reaction, the esters subjected to the cleavage reaction are hydrolyzed to yield a carboxylic acid salt and an alcohol.

As a base, conventionally known compounds can be used which are able to provide hydroxy ions (OH⁻) in an aqueous solution. In the context of the invention, metal hydroxides or metal oxides are preferably used as a base, and NaOH and KOH are particularly preferred examples of such bases.

In the aqueous base, the base is typically completely dissolved in water at the reaction temperature of the ester cleavage reaction, but it is also possible to use a saturated solution of the base wherein a part of the base remains undissolved. If an undissolved base is present at the start of the reaction, it may still take part in the reaction when the base is consumed while the cleavage of the ester proceeds. However, it is preferred that the aqueous base used for the ester cleavage reaction represents a solution of the base wherein the base is completely dissolved at the reaction temperature of the ester cleavage reaction, i.e. that it represents an aqueous base wherein no base remains undissolved.

In the aqueous base which is used to carry out the ester cleavage reaction, the concentration of the base in water is 20 wt% or more, preferably 25 wt% or more, more preferably 30 wt% or more, and still more preferably 35 wt% or more. The concentration in wt% as referred to herein indicates the concentration of the dissolved base in relation to the total weight of the dissolved base and the water in the aqueous base. It was found that such concentrations and preferred concentrations allow the ester cleavage reaction to proceed with advantageously high yields that can be achieved during short periods of time for the reaction.

The amount of the aqueous base which is used for the ester cleavage reaction can be suitably selected depending on the concentration of the base and on the amount of the ester to be hydrolyzed in the ester cleavage reaction. Generally, the amount of the aqueous base used in the ester cleavage reaction is such that 1.0 molar equivalents of the base or more, preferably 1.1 molar equivalents of the base or more, are available per mole of ester to be hydrolyzed in the ester cleavage reaction. Typically, available amounts of more than 1.3 molar equivalents of the base will not lead to an additional benefit for the reaction, such that available amounts of 1.0 molar equivalents to 1.3 molar equivalents are more preferred, and available amounts of 1.1 molar equivalents to 1.3 molar equivalents are even further preferred.

As will be appreciated by the skilled reader, the amount of base that is available for the hydrolysis of the ester may be affected by acidic components that can optionally be provided in the starting material together with the one or more esters that are subjected to the ester cleavage reaction. For example, free carboxylic acids may be present in a wax used as a starting material, or acidic components may be set free in the starting material under the conditions of the reaction. Such acidic components may neutralize (or may be neutralized by) a portion of the base that is added to the starting material, so that this portion of the base will no longer be available for the ester cleavage reaction. Thus, it is preferable to use the aqueous base (i) in an amount of 1.0 molar equivalents of the base or more, more preferably 1.1 molar equivalents of the base or more, per mole of ester to be hydrolyzed in the ester cleavage reaction, and (ii) an additional amount of base which is sufficient to neutralize acidic components optionally provided in the starting material. Also in this context, more than 1.3 molar equivalents per mole of ester to be hydrolyzed are typically not needed in (i). Furthermore, it will be understood that, if no such acidic components are provided in the starting material, the sufficient amount referred to in (ii) will be 0.

It is particularly preferred that the aqueous base is used (i) in an amount of 1.0 molar equivalents of the base or more, more preferably 1.1 molar equivalents of the base or more, per mole of ester that is contained in the starting material, and (ii) an additional amount of base which is sufficient to neutralize acidic components optionally provided in the starting material. Also in this context, more than 1.3 molar equivalents per mole of ester contained in the starting material are typically not needed in (i).

In line with the above, it will be understood that a preferred embodiment of the present invention is a process for the oxidation of an ester which is contained in a wax (also referred to as a wax ester) via an ester cleavage reaction, followed by the oxidation of the alcohol component of the ester, to provide an oxidation product of the wax, said process comprising a step of subjecting a wax as a starting material comprising one or more esters of a carboxylic acid having a carbon number of 14 or more with an alcohol having a carbon number of 14 or more to an ester cleavage reaction wherein at least a part of the one or more esters is hydrolyzed to obtain a mixture of one or more carboxylic acid salts with a carbon number of 14 or more and one or more alcohols with a carbon number of 14 or more, wherein the ester cleavage reaction is carried out under atmospheric pressure and in a temperature range of 90 to 140 °C by exposing the esters comprised in the wax to an aqueous base with a concentration of the base in water of 20 wt% or more, more preferably 25 wt% or more,
and wherein the aqueous base is used in an amount such that 1.0 molar equivalents of the base or more, preferably 1.1 molar equivalents of the base or more, are available per mole of ester to be hydrolyzed in the ester cleavage reaction;
a step of acidifying the one or more carboxylic acid salts with a carbon number of 14 or more to obtain one or more carboxylic acids; and
a step of oxidizing at least a part of the one or more alcohols with a carbon number of 14 or more to obtain one or more carboxylic acids;
wherein the step of acidifying the carboxylic acid salts and the step of oxidizing the alcohols can be carried out concurrently or successively.

The starting material containing the esters and the aqueous base are combined in a reaction mixture in order to carry out the ester cleavage reaction by exposing the esters to the aqueous base with a concentration of the base in water as defined herein.

Various approaches will be available to the skilled person in order to provide a reaction mixture of the starting material comprising the esters and the aqueous base, and to carry out the ester cleavage reaction. For example, the aqueous base may be combined with the starting material in one portion, or may be added to the starting material batchwise or continuously over a certain period of time. Preferably, when the starting material and the aqueous base are combined, the starting material is kept at a temperature where it forms a liquid phase, i.e. the esters to be subjected to the ester cleavage reaction are in a molten state. The aqueous base may be combined with the starting material while it is kept at room temperature (e.g. 20°C). However, in order to achieve a high concentration of base which is dissolved in the water of the aqueous base, it may be advantageous to provide the aqueous base at temperatures above room temperature to combine it with the starting material.

Moreover, in order to provide the reaction mixture of the starting material comprising the esters and the aqueous base, it is possible to combine the starting material with an aqueous base, i.e. a base dissolved in water, or to form the aqueous base *in situ,* e.g. by first combining the starting material comprising the esters with water, and subsequently adding a base. Similarly, if desired, the concentration of the aqueous base can be adjusted by combining the starting material with an aqueous base at a higher concentration of the base, and adjusting the concentration within the limits defined above by adding additional water to the mixture. It is also possible to combine the starting material with an aqueous base at a lower concentration and to increase the concentration of the base to a desired concentration of the base in water as defined herein by evaporating water. However, it is preferred that the starting material is combined with an aqueous base having a concentration of the base in water as defined herein, so that the esters contained in the starting material are exposed to an aqueous base with a suitable concentration of the base directly at the start of the ester cleavage reaction.

Advantageously, in order to carry out the process in accordance with the present invention, the use of organic solvents is not necessary, and the ester cleavage reaction proceeds efficiently under the conditions described above in the absence of an organic solvent. Thus, the ester cleavage reaction is preferably carried out in the absence of an organic solvent. In this regard, it will be understood that a solvent, as referred to herein, is a substance which is liquid below the melting point of the starting material comprising the one or more esters and at atmospheric pressure (e.g. 1013.25 hPa) and which is used in a reaction to dissolve or disperse one or more of the reactants. In other words, the starting material comprising the one or more esters, which is typically a solid or a soft solid at room temperature and atmospheric pressure is obviously not encompassed by the organic solvents as referred to above. As will be understood by the skilled person, the melting point of the starting material comprising the one or more esters is the lowest temperature at which the starting material forms a liquid phase (i.e. a molten liquid phase).

Under the conditions described above, the ester cleavage reaction can be efficiently carried without the need for a pressurization of the reaction mixture. Thus, the reaction is carried out under atmospheric pressure.

The ester cleavage reaction of the process in accordance with the invention is preferably carried out over a period of 10 h or less, preferably 5 h or less, and more preferably 3 h or less.

Since the ester cleavage reaction proceeds efficiently under the conditions described above, conversion rates of 70 % or more, advantageously 80 % or more, more preferably 90 % or more, and still more preferably 95 % or more can be achieved, even when using limited reaction times as mentioned above. The conversion rate is indicated as the molar percentage of esters that is hydrolyzed during the ester cleavage reaction, based on the molar amount of esters contained in the starting material.

Subsequently to the ester cleavage reaction, the process in accordance with the invention comprises a step of acidifying the one or more carboxylic acid salts with a carbon number of 14 or more which result from the ester cleavage reaction to obtain one or more carboxylic acids.

This step can be carried out, e.g., by adding to the reaction mixture resulting from the ester cleavage reaction an acid or an aqueous solution of an acid. Suitable acids include, for example, sulfuric acid, hydrochloric acid, formic acid, acetic acid, propionic acid or citric acid. The concentration of the acid in the aqueous solution can be adjusted, for example, to 10 wt% or more, e.g. 20 or 50 wt.%, or the acid can be added as a pure substance. Preferably the acid or acidic solution is liquid when added. Diluted acids are more preferred for the addition than pure acids, in order to make sure that no (local) re-esterification and uncontrolled oxidations (e.g. with sulfuric acid) occur. The degree of dilution may be chosen to fit the preferred follow-up reactions and work-up procedures and is not restricted by chemical reasons. Acid concentrations between 5 and 50 wt.% proved to be easily manageable, e.g. for laboratory implementations of up to 1 kg batch reactions, without inflicting re-esterification or other unwanted reactions.

In a further step of the process in accordance with the present invention, at least a part of the one or more alcohols which are provided by the ester cleavage reaction is oxidized to obtain one or more carboxylic acids.

The oxidation reaction to which at least a part of the alcohols provided by the oxidation reaction is subjected during the process of the present invention leads to the oxidation of the alcohols to carboxylic acids. The alcohols may be quantitatively (i.e. completely) oxidized to carboxylic acids, or, if desired, the oxidation reaction may be controlled such that only a part of the alcohols is oxidized. In that case, the material resulting from the oxidation reaction will still contain a part of the alcohols that have been provided during the ester cleavage reaction.

It is possible to separate the alcohols which are provided by the ester cleavage reaction from other products resulting from the ester cleavage reaction prior to the step of oxidizing the alcohols. However, typically the alcohols which are provided by the ester cleavage reaction are oxidized in the presence of the carboxylic acid salts or the carboxylic acids obtained from the ester cleavage reaction or the ester cleavage reaction and the acidification step. Thus, the process in accordance with the invention is typically used to provide a product containing the carboxylic acids which are obtained from the ester cleavage reaction and the acidification together with the carboxylic acids resulting from the oxidation of the alcohols.

The oxidation of the alcohols can be achieved by known methods, such as an oxidation using a transition metal or a transition metal compound as an oxidizing reagent. For example, the oxidation reaction may be carried out using a Cr(VI) compound, preferably using chromosulfuric acid. Following this latter approach, the required amount of Cr(VI) for the oxidation of the esters is still significantly reduced compared to an oxidation process wherein the ester is reacted directly with chromosulfuric acid. For example, in experimental data in the application US 2015/0284661 A1 4960 mL of chromosulfuric acid (100g CrO₃/L) were used to oxidize 500 g of saponified rice wax (~1 g(CrO₃)/1 g(rice wax)), whereas in the process in accordance with the present invention, sufficient oxidation was achieved with 0.20 g(CrO₃)/1 g (rice wax). This results in app. 80 % reduction in the amount of used chromium compounds. Theoretically 1.334 mol Cr(VI) compound are needed to oxidize one mol alcohol (resulting from the hydrolyzed ester). To attain fast and reliable oxidation, we find it preferable to use slightly higher amounts of 1.4 mol Cr(VI) or more per mol alcohol. Due to economic and environmental reason the use of 3.0 mol Cr(VI) or less, more preferably 2.0 mol Cr(VI) or less is advised for this method.

Preferably, the alcohols provided by the ester cleavage reaction are subjected to an oxidation reaction which does not use chromium containing reactants.

It is particularly preferred in the process in accordance with the present invention that the step of oxidizing at least a part of the one or more alcohols with a carbon number of 14 which are provided by the ester cleavage reaction to obtain one or more carboxylic acids comprises the oxidation of the alcohols using an oxoammonium cation and a co-oxidant as oxidizing agents at a reaction temperature of 50°C or higher to obtain the carboxylic acids. This preferred variant of the step of oxidizing the one or more alcohols is also referred to as the "preferred oxidation step" herein.

Such an oxidation reaction allows the oxidation of the alcohols to be achieved under environmentally acceptable and mild conditions with high yields and without undesirable side reactions, such as the formation of esters or a cleavage of carbon-carbon bonds in the alcohols subjected to the oxidation reaction. It can be particularly favorably applied if the alcohols with a carbon number of 14 or more to be oxidized are primary alcohols.

In line with the above, it will be understood that a further preferred embodiment of the present invention is a process for the oxidation of an ester which is contained in a wax via an ester cleavage reaction, followed by the oxidation of the alcohol component of the ester, to provide an oxidation product of the wax, said process comprising:
a step of subjecting a wax as a starting material comprising one or more esters of a carboxylic acid having a carbon number of 14 or more with an alcohol having a carbon number of 14 or
more to an ester cleavage reaction wherein at least a part of the one or more esters is hydrolyzed to obtain a mixture of one or more carboxylic acid salts with a carbon number of 14 or more and one or more alcohols with a carbon number of 14 or more, wherein the ester cleavage reaction is carried out under atmospheric pressure and in a temperature range of 90 to 140 °C by exposing the esters comprised in the wax to an aqueous base with a concentration of the base in water of 20 wt% or more, more preferably 25 wt% or more,
and wherein the aqueous base is used in an amount such that 1.0 molar equivalents of the base or more, preferably 1.1 molar equivalents of the base or more, are available per mole of ester to be hydrolyzed in the ester cleavage reaction;
a step of acidifying the one or more carboxylic acid salts with a carbon number of 14 or more to obtain one or more carboxylic acids; and
a step of oxidizing at least a part of the one or more alcohols with a carbon number of 14 which are provided by the ester cleavage reaction to obtain one or more carboxylic acids, comprising the oxidation of the alcohols using an oxoammonium cation and a co-oxidant as oxidizing agents at a reaction temperature of 50°C or higher to obtain the carboxylic acids;
wherein the step of acidifying the carboxylic acid salts and the step of oxidizing the alcohols can be carried out concurrently or successively.

As will be understood be the skilled reader, this preferred oxidation step may be accomplished using one single type of an oxoammonium cation, or two or more types of oxoammonium cations differing in their structure. Similarly, the oxidation reaction may be accomplished using one single type of co-oxidant, or two or more types of co-oxidants differing in their structure.

The use of oxoammonium cations as oxidation catalysts is known in the art (e.g. R. Ciriminna et al., Organic Process Research & Development, 2010, 14, 245-251 or M. Shibuya et al., J. Am. Chem. Soc., 2006, 128, 8412-8413), and the oxoammonium cation may be generated *in situ* for this purpose using an corresponding aminoxyl radical or hydroxylamine as a precursor compound.

Suitable oxoammonium cations which can also be employed in the preferred oxidation step in accordance with the invention typically have a structure which can be schematically represented as R₂N⁺=O, wherein the two groups R are independently an organic group bound via a secondary or tertiary carbon atom (i.e. a carbon atom bound to two or three further carbon atoms) to the nitrogen, and wherein the two groups R may be combined to form a heterocyclic ring together with the nitrogen atom to which they are bound. The anion which acts as a counter-ion for the cationic oxoammonium ion is not particularly limited. Examples are halide ions, such as chloride or bromide, and hydroxy ions, and preferred are chloride or bromide.

Preferred as oxoammonium cation for use in the preferred oxidation step in accordance with the invention is an oxoammonium cation selected from a 2,2,6,6-tetramethylpiperidine-oxoammonium cation and from derivatives thereof carrying a substituent at the 4-position of the piperidine ring, from a 9-azabicyclo[3.3.1]nonane-oxoammonium cation, and from a 2-azaadamantane-oxoammonium cation, a 1-methyl-2-azaadamantane-oxoammonium cation and a 1,3-dimethyl-2-azaadamantane-oxoammonium cation.

Thus, a further preferred oxoammonium cation for use in the preferred oxidation step in accordance with the invention is an oxoammonium cation having a structure as shown in the following formulae (Ia) to (IIIa):

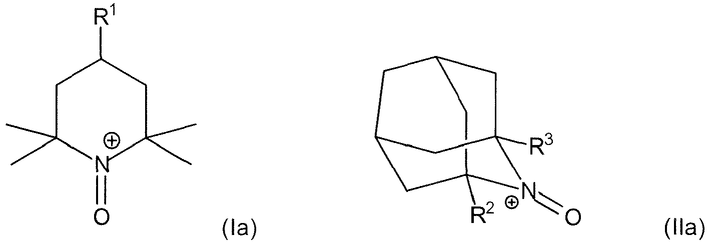

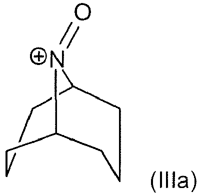

In formula (Ia), R¹ is selected from hydrogen, -OH, =O, -OR⁴, -NH₂, and -NHC(O)R⁵. R⁴ designates an alkyl group, an aryl group, an aralkyl group or a group -C(O)R⁶, with R⁶ being selected from a C1-C6 alkyl group and a phenyl group. The alkyl group is preferably a C1-C6 alkyl group, the aryl group is preferably a phenyl group and the aralkyl group is preferably a benzyl group. More preferably, R⁴ designates a C1-C6 alkyl group, and most preferably a methyl group. R⁵ designates a C1-C6 alkyl group, and preferably a methyl group. It will be understood that if R¹ is =O, the carbon atom to which R¹ is attached carries no hydrogen atom.

In formula (IIa), R² and R³ are independently selected from hydrogen and methyl.

The oxoammonium cation can be added to the reaction mixture in the form of the oxoammonium salt, or can be generated *in situ,* i.e. in the reaction mixture for the oxidation reaction, from a suitable precursor compound, for example from an aminoxyl radical or from a hydroxylamine. Preferably, the precursor compound is an aminoxyl radical. Thus, in accordance with a further preferred variant of preferred oxidation step, the step of oxidizing at least a part of the one or more alcohols with a carbon number of 14 which are provided by the ester cleavage reaction to obtain one or more carboxylic acids comprises providing a reaction mixture which comprises (i) the one or more alcohols with a carbon number of 14 and (ii) a precursor compound for an oxoammonium cation, and (iii) a co-oxidant,
allowing the precursor compound to form an oxoammonium cation, and oxidizing the alcohols using the oxoammonium cation and the co-oxidant as oxidizing agents at a reaction temperature of 50°C or higher to obtain one or more carboxylic acids.

As will be understood by the skilled reader, the reaction mixture as referred to above would be provided and the oxoammonium cation would be allowed to form before the oxidation reaction takes place to which the alcohols are subjected in the preferred oxidation step.

In order to provide an oxoammonium cation as discussed above, an aminoxyl radical which may be used as a precursor compound in the in the preferred oxidation step is typically an aminoxyl radical having the structure R₂N-O•, wherein the dot at the oxygen atom indicates an unpaired electron, and wherein the two groups R are independently an organic group bound via a secondary or tertiary carbon atom to the nitrogen, and wherein the two groups R may form a heterocyclic ring together with the nitrogen atom to which they are bound.

More preferably, the aminoxyl radical which may be used in the preferred oxidation step is selected from 2,2,6,6-tetramethylpiperidine-1-oxyl (TEMPO) and from derivatives thereof carrying a substituent at the 4-position of the piperidine ring, from 9-azabicyclo[3.3.1]nonane N-oxyl (ABNO), and from 2-azaadamantane N-oxyl (AZADO), 1-methyl-2-azaadamantane N-oxyl (1-Me-AZADO) and 1,3-dimethyl-2-azaadamantane N-oxyl (1,3-dimethyl-AZADO).

As suitable 4-substitued TEMPO derivatives, the following exemplary compounds can be specifically mentioned for use in the context of the present invention: 4-OH-TEMPO, 4-Oxo-TEMPO, 4-methoxy-TEMPO (4-MeO-TEMPO), 4-NH₂-TEMPO, 4-acetamido-TEMPO (4-AA-TEMPO), 4-carboxy-TEMPO, and 4-hydroxy-TEMPO benzoate.

Thus, a further preferred aminoxyl radical which may be used in the preferred oxidation step is an aminoxyl radical having a structure as shown in the following formulae (Ib) to (IIIb):

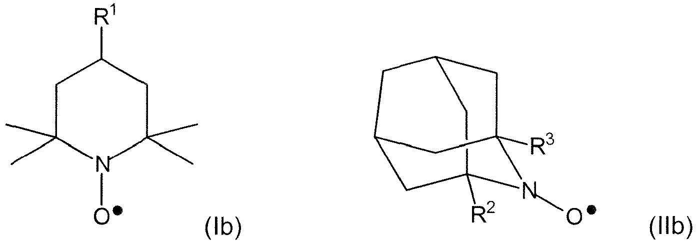

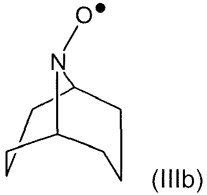

In formula (Ib), R¹ is selected from hydrogen, -OH, =O, -OR⁴, -NH₂, and -NHC(O)R⁵. R⁴ designates an alkyl group, an aryl group, an aralkyl group or a group -C(O)R⁶, with R⁶ being selected from a C1-C6 alkyl group and a phenyl group. The alkyl group is preferably a C1-C6 alkyl group, the aryl group is preferably a phenyl group and the aralkyl group is preferably a benzyl group. More preferably, R⁴ designates a C1-C6 alkyl group, and most preferably a methyl group. R⁵ designates a C1-C6 alkyl group, and preferably a methyl group. It will be understood that if R¹ is =O, the carbon atom to which R¹ is attached carries no hydrogen atom.

In formula (IIb), R² and R³ are independently selected from hydrogen and methyl.

The aminoxyl radical as discussed above may also be used in the preferred oxidation step in immobilized form on a carrier, e.g. supported by a carrier without chemical bonds between the carrier and the aminoxyl radical, or covalently bound to a carrier. Preferred carriers are a silica gel or a polymer. In this regard, TEMPO and its 4-substituted derivatives, such as 4-OH-TEMPO and 4-NH₂-TEMPO are particularly useful. The substituent in 4-position may act as a functional group for the formation of a bond with the carrier (e.g. an ether bond, an ester bond, an amino bond or an amide bond).

Particularly preferred as aminoxyl radicals which may be used in the preferred oxidation step are 4-OH-TEMPO, 4-NH₂-TEMPO, 4-MeO-TEMPO, 4-AA-TEMPO, TEMPO, Me-AZADO, ABNO, or any of these aminoxyl radicals supported on or bound to a carrier. Most preferred in terms of a balance between costs and efficiency is 4-OH-TEMPO as aminoxyl radical.

An aminoxyl radical can be conveniently used as a precursor compound of an oxoammonium cation, since aminoxyl radical compounds are commercially available and can form an oxoammonium cation spontaneously via disproportionation of the radical. However, the formation of the cation may also be accelerated by adding a component for the acceleration of the reaction as discussed below.

As far as examples of hydroxylamines are concerned which are suitable as precursor compounds in the preferred oxidation step, the above information regarding the general and preferred structures of suitable aminoxyl radicals equally applies, except for the fact that the hydroxylamine carries an -OH or -OR substituent attached to the nitrogen atom instead of a radical -O•. In these hydroxylamines, R is a hydrocarbon group such as an alkyl group, an aryl group or an aralkyl group. The alkyl group is preferably a C1-C6 alkyl group, the aryl group is preferably a phenyl group and the aralkyl group is preferably a benzyl group. However, if a hydroxylamine is used as as a precursor compound of an oxoammonium cation, the hydroxylamine is preferably a compound which carries an -OH substituent instead of a radical -O• attached to the nitrogen atom of the aminoxyl radicals and preferred aminoxal radicals discussed above.

Without wishing to be bound by theory, it is assumed that the oxoammonium cation can act as a catalytically active species in the oxidation reaction. In this reaction, the oxoammonium cation is reduced to a hydroxylamine while the alcohol is oxidized. In the presence of a co-oxidant which can oxidize the hydroxylamine, the oxoammonium cation can be recovered.

In the preferred oxidation step that may be carried out in the process of the present invention, the oxoammonium cation or a precursor thereof, such as an aminoxyl radical or a hydroxylamine, is typically combined with the alcohols in order to carry out the oxidation reaction in an amount of 0.01 mol% or more, preferably 0.1 mol% or more, more preferably 0.2 mol% or more, and still more preferably 0.5 mol% or more, based on the total number of moles of alcohol group to be oxidized. When the amount of oxoammonium cation or precursor thereof is increased, the yield of carboxylic acid and/or the rate of the reaction may increase. However, when a certain amount is exceeded, no further improvements in terms of an increased yield or reaction rate are observed. Moreover, the costs of the process are higher with a higher amount of aminoxyl radical. Thus, the oxoammonium cation or a precursor thereof is typically combined with the alcohols in the preferred process variant in accordance with the invention in amounts of not more than 20 mol%, preferably not more than 10 mol%, still more preferably not more than 5 mol%, and even more preferably not more than 3 mol%, based on the total number of moles of alcohol group to be oxidized.

A further component which is used in the preferred oxidation step is the co-oxidant. An effective co-oxidant for use in the process of the present invention is preferably selected from a chlorite (i.e. a salt with the anion ClO₂⁻) and a bromite (i.e. a salt with the anion BrO₂⁻). More preferably, a chlorite is used.

Suitable cations for the chlorite and the bromite include, e.g., an ammonium cation and alkali cations such as Na⁺ and K⁺. In terms of costs and availability, sodium chlorite is a preferred co-oxidant.

In the preferred oxidation step, the amount of the co-oxidant which is combined with the alcohols in order to subject them to the oxidation reaction can be suitably chosen depending on the desired rate of the oxidation reaction. For example, if only a part of the alcohols is to be oxidized, an amount of the co-oxidant of less than 1 molar equivalent (eq.) of co-oxidant per mole of alcohol groups can be used, e.g. 0.75 eq. For a complete oxidation, the co-oxidant should be used in an amount of 1.0 molar equivalents or more, preferably in a slight excess compared to alcohol groups to be oxidized. Thus, the co-oxidant is preferably used in an amount of 0.75 molar equivalents or more, more preferably 1.0 molar equivalents or more, even more preferably 1.05 molar equivalents or more, per mole of alcohol group. The maximum amount is not particularly limited, but amounts of more than 1.5 molar equivalents in general do not give rise to advantages and increase the costs of the reaction. Thus, the co-oxidant is preferably used in an amount of 1.5 molar equivalents or less, more preferably 1.3 molar equivalents or less, per mole of alcohol group.

Especially in cases where a precursor compound of an oxoammonium cation, such an an aminoxyl radical, is combined with the alcohols to be oxidized, the start of the oxidation reaction may be accelerated by additionally adding a component selected from a hypochlorite (i.e. a salt with the anion OCl⁻), a hypobromite (i.e. a salt with the anion OBr⁻), chlorine gas or any chemical that in-situ produces one of these substances, e.g., trichloroisocyanuric acid, or an aldehyde in combination with NaClO₂. Among these, hypochlorite and hypobromite are preferred; and the hypochlorite is particularly preferred. Suitable cations for the hypochlorite and the hypobromite include, e.g., an ammonium cation and alkali cations such as Na⁺ and K⁺. In terms of costs and availability, sodium hypochlorite is a preferred optional component for the acceleration of the reaction.

If such a component for the acceleration of the reaction is used, it is typically used in amounts of 0.2-5.0 mol% per mole of alcohol group to be oxidized. Moreover, it is preferred that the amount of the component for the acceleration of the reaction is used in a molar ratio in the range of 1-2 with regard to the amount of the precursor compound of the oxoammonium cation.

During the preferred oxidation step in accordance with the present invention, the use of an organic solvent is not necessary. Thus, both the step of subjecting a starting material comprising one or more esters to an ester cleavage reaction and the step of oxidizing at least a part of the resulting alcohols are preferably carried out in the absence of an organic solvent as discussed above.

On the other hand, the preferred oxidation step comprising the use of an oxoammonium cation and a co-oxidant as oxidizing agents is preferably carried out in the presence of an aqueous medium. As will be understood by the skilled reader, an aqueous medium is a medium formed by water as a solvent, wherein one or more components may be dissolved or dispersed. In the preferred case where an aqueous medium is present during the oxidation reaction in the process according to the invention, a significant portion of the oxoammonium cation and of the co-oxidant acting as oxidizing agents can be contained in the aqueous medium. In order to provide the reaction mixture for the preferred oxidation step, the oxoammonium cation or a precursor compound thereof, the co-oxidant and the optional component for the acceleration of the reaction may, e.g., be dissolved in one or more aqueous media prior to the oxidation reaction and added to the alcohols or to a mixture of the alcohols and an aqueous medium prior to the oxidation reaction.

Moreover, in the preferred case where an aqueous medium is present during the preferred oxidation step, the aqueous medium is preferably adjusted to a pH of 8 or less, more preferably a pH of 6 or less. Typically, an acid or a buffer is present in the aqueous medium in order to adjust the pH thereof. Exemplary suitable acids include HCl, H₃PO₄, citric acid and acetic acid. Exemplary suitable buffers include an acetic acid/acetate buffer.

In the preferred case where an aqueous medium is present during the preferred oxidation step, the aqueous medium may form a separate phase in the reaction mixture of the oxidation step in view of the fact that the alcohols with a carbon number of 14 or more typically show hydrophobic characteristics. Depending on the relative volume ratio of the aqueous medium and of the organic phase, the aqueous medium may form a continuous phase during the oxidation step wherein the organic phase is dispersed, or the organic phase may form a continuous phase wherein the aqueous phase is dispersed. As will be understood, the organic phase in the preferred oxidation step comprises the alcohols to be oxidized and/or the carboxylic acids which result from the oxidation of the alcohols, and typically further comprises the carboxylic acid salts or the carboxylic acids obtained from the ester cleavage reaction or the ester cleavage reaction and the acidification step.

The volume ratio of the organic phase comprising the alcohols to be oxidized to the aqueous medium is not particularly limited. Typical volume ratios range from 10:1 , preferably 5:1 , more preferably 3:2, to 1 : 10, preferably 1 :5 (indicated as volume organic phase:volume aqueous medium at the reaction temperature of the preferred oxidation step and at the start of the oxidation reaction).

The reaction temperature for the oxidation reaction in the preferred oxidation step is 90°C or higher. Usually, the temperature is adjusted such that the organic phase comprising the alcohols to be oxidized is softened or molten, preferably molten, so that the reactants forming the reaction mixture can be conveniently mixed. Thus, the oxidation reaction is preferably carried out at a temperature where the organic phase comprising the alcohols to be oxidized is in a liquid form (i.e. a molten liquid form). This temperature may be a temperature higher than the melting points of the individual components in the organic phase. However, since a mixture of organic compounds may have a melting point which is lower than the individual melting points of the components of the mixture, a molten liquid may already exist at a temperature which is lower than the lowest individual melting point of the components of the organic phase.

Generally, temperatures of more than 150°C are not needed, and temperatures below 100°C are preferred e.g. for economic reasons.

As will be understood by the skilled reader, the reaction temperature should not exceed the boiling point of any of the components used in the reaction at the pressure at which the reaction is carried out. The reaction can be conveniently carried out at atmospheric pressure. In the preferred case where an aqueous medium is present during the oxidation reaction in the process according to the invention, the reaction temperature should thus be less than 100°C if the reaction is carried out at atmospheric pressure.

In order to carry out the oxidation reaction using the preferred oxidation step, an intermediate material comprising the one or more alcohols to be oxidized, the oxoammonium cation or a precursor compound thereof, the co-oxidant and any optional components which may be used in the reaction, are combined. As noted above, the alcohols which are provided by the ester cleavage reaction are typically oxidized in the presence of the carboxylic acid salts or the carboxylic acids obtained from the ester cleavage reaction or the ester cleavage reaction and the acidification step, so that carboxylic acid salts or the carboxylic acids are typically contained in the intermediate material as well.

Typically, the intermediate material comprising the one or more alcohols and the oxoammonium cation or a precursor compound thereof are combined by mixing the intermediate material with the oxoammonium cation or a precursor compound thereof. This step can be conveniently carried out by mixing the oxoammonium cation or a precursor compound thereof as a solution in an aqueous medium with the intermediate material. Mixing is typically carried out at an increased temperature, i.e. a temperature as it is discussed as the reaction temperature for the oxidation temperature above. At a temperature where the intermediate material for the oxidation reaction is in a liquid form (i.e. a molten liquid form), the components of the reaction can be conveniently mixed by stirring. As will be understood, the intermediate material and the oxoammonium cation or a precursor compound thereof, preferably as a solution in an aqueous medium, can e.g. be combined at a lower temperature, followed by an increase in temperature and mixing. Alternatively, the intermediate material and the oxoammonium cation or a precursor compound thereof, preferably as a solution in an aqueous medium, can be combined under stirring at an increased temperature.

The co-oxidant can be combined with the intermediate material comprising the one or more alcohols prior to, during or after the intermediate material has been combined with the oxoammonium cation or a precursor compound thereof. Also this step can be conveniently carried out by adding and mixing the co-oxidant as a solution in an aqueous medium with the intermediate material comprising one or more alcohols, or with the intermediate material and the oxoammonium cation or a precursor compound thereof. Preferably, the co-oxidant is added and mixed as a solution in an aqueous medium with a mixture comprising the intermediate material and the oxoammonium cation or a precursor compound thereof, typically over a certain period of time, e.g. over 10 min to 5 hours, preferably 30 min to 3 hours. Also the mixing of the co-oxidant with the intermediate material or with the intermediate material and the oxoammonium cation or a precursor compound thereof is typically carried out at an increased temperature, i.e. a temperature as it is discussed as the reaction temperature for the oxidation temperature above. Co-oxidant and oxoammonium cation or a precursor compound thereof may also be added simultaneously at once or over a certain period of time, e.g. over 10 min to 5 hours, preferably 30 min to 3 hours.

Other components for the reaction, such as the optional component for the acceleration of the reaction, or additional components of an aqueous medium may be added as needed or as convenient. For example, these components may be contained as a component in an aqueous medium in which the aminoxyl radical is dissolved, and/or in an aqueous medium wherein the co-oxidant is dissolved.

Once the desired amounts of the oxoammonium cation or its precursor compound, of the co-oxidant and of any further optional components have been combined with the intermediate material, the oxidation reaction is typically allowed to proceed for a certain period of time at the reaction temperature of the oxidation reaction discussed above. The reaction time for the oxidation reaction can be appropriately selected by the skilled person. Typically, it ranges from 1 to 10 hours, preferably from 1 to 5 hours.

As a result of the oxidation step, at least a part of the alcohols provided by the ester cleavage reaction is oxidized to the corresponding carboxylic acids. As will be understood by the skilled reader, a carboxylic acid corresponding to an alcohol is one wherein the carbon number of the carboxylic acid is the same as that of the alcohol which has undergone the oxidation reaction. Typically, 70 mol% or more, preferably 80 mol% or more, and even more preferably 90 mol% or more of the alcohol groups in the alcohols provided by the ester cleavage reaction (based on the total number of alcohol groups as 100 mol%) will be converted to a carboxylic acid group or a salt thereof as a result of the oxidation step of the process in accordance with the invention.

In line with the above, it will be understood that a still further preferred embodiment of the present invention is a process for the oxidation of an ester which is contained in a wax (also referred to as a wax ester) via an ester cleavage reaction, followed by the oxidation of the alcohol component of the ester, to provide an oxidation product of the wax, said process comprising
a step of subjecting a wax as a starting material comprising one or more esters of a carboxylic acid having a carbon number of 14 or more with an alcohol having a carbon number of 14 or more to an ester cleavage reaction wherein at least a part of the one or more esters is hydrolyzed to obtain a mixture of one or more carboxylic acid salts with a carbon number of 14 or more and one or more alcohols with a carbon number of 14 or more,
wherein the ester cleavage reaction is carried out under atmospheric pressure and in a temperature range of 90 to 140 °C by exposing the esters comprised in the wax to an aqueous base with a concentration of the base in water of 20 wt% or more, more preferably 25 wt% or more,
and wherein the aqueous base is used in an amount such that 1.0 molar equivalents of the base or more, preferably 1.1 molar equivalents of the base or more, are available per mole of ester to be hydrolyzed in the ester cleavage reaction;
a step of acidifying the one or more carboxylic acid salts with a carbon number of 14 or more to obtain one or more carboxylic acids;
a step of oxidizing at least a part of the one or more alcohols with a carbon number of 14 to obtain one or more carboxylic acids, comprising
providing a reaction mixture which comprises (i) the one or more alcohols with a carbon number of 14 and (ii) an aminoxyl radical as a precursor compound for an oxoammonium cation, and (iii) a co-oxidant,
allowing the precursor compound to form an oxoammonium cation, and oxidizing the alcohols using the oxoammonium cation and the co-oxidant as oxidizing agents at a reaction temperature of 50°C or higher to obtain the carboxylic acids,
wherein the aminoxyl radical is an aminoxyl radical having the structure R₂N-0•, wherein the dot at the oxygen atom indicates an unpaired electron, and wherein the two groups R are independently an organic group bound via a secondary or tertiary carbon atom to the nitrogen, and wherein the two groups R form a heterocyclic ring together with the nitrogen atom to which they are bound, and is more preferably selected from 4-OH-TEMPO, 4-NH₂-TEMPO, 4-MeO-TEMPO, 4-AA-TEMPO, TEMPO, Me-AZADO, ABNO, and from any of these aminoxyl radicals supported on or bound to a carrier,
the co-oxidant is a chlorite,
and optionally a hypochlorite is additionally added in the step of oxidizing the alcohols; and wherein the step of acidifying the carboxylic acid salts and the step of oxidizing the alcohols can be carried out concurrently or successively.

### Examples

In the case of C₁₆-O₂C₁₈, all selectivities S, conversions X and yields Y are given in respect to molar contributions.

The conversion of esters (s) (X [%(I_{Monomeres}/I_{Total})] and the ester ratio [%(I)] after oxidation are calculated with the integrals obtained by GC analyses.

### Ester Cleavage Reaction

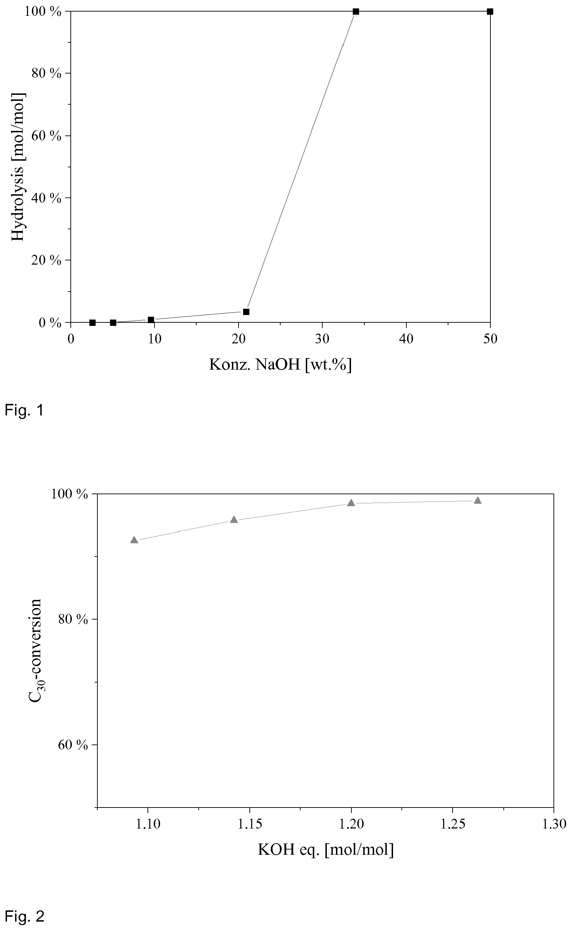

Procedure 1: 0.2 g C₁₆-O₂C₁₈ and the amount NaOH necessary to attain a solution of the specified concentration dissolved with 2 mL H₂O (cf. Table 1) were added into the reaction vessel, heated to 110 °C and stirred for 4 hours. Diagram 1 (Fig. 1) shows a summary of the results.

**Table 1: Experiments according to Procedure 1**

| | **NaOH conc.** | **X** |
|---|---|---|
| | **[wt.%]** | **[%]** |
| 1 | 2.6 | 0.0 |
| 2 | 5.0 | 0.0 |
| 3 | 9.6 | 1.0 |
| 4 | 20.9 | 3.5 |
| 5 | 34.0 | 100 |
| 6 | 50.0 | 100 |

Procedure 2: 1.0 g of RBW (rice bran wax) and the specified amount of 42 wt.% KOH as aqueous solution were added into the reaction vessel, heated to 110 °C and stirred for 3 hours. Diagram 2 (Fig. 2) shows a summary of the results.

**Table 2: Experiments according to Procedure 2.**

| **#** | **Eq. of base [mol/mol]** | **Amount of base* [mg]** | **H₂O amount at beginning [mg]** | **X [%(I_{Monomeres}/I_{Total})]** |
|---|---|---|---|---|
| 7 | 1.09 | 95.6 | 95.6 | 92.5 |
| 8 | 1.13 | 100.0 | 100.0 | 95.5 |
| 9 | 1.21 | 104.9 | 104.9 | 98.5 |
| 10 | 1.26 | 110.4 | 110.4 | 99.0 |

| | | | | |
|---|---|---|---|---|
| * KOH had a purity of 85 % | | | | |

### Experiment 11:

50.0 g RBW were heated to 110 °C. 3.555 g NaOH were dissolved in 10 mL H₂O (resulting in a 26 wt.% solution) and slowly added to the stirred, molten wax. Foam was forming, indicating the exothermic hydrolysis of the wax. The mixture became highly viscous after 10 minutes and it is assumed that most of the wax had reacted at this point. Water was added to lower the viscosity and to secure efficient stirring. 40 minutes after the reaction start 70 mL of water had been added and the temperature of the reaction was measured to be 96 °C. 60 minutes after the reaction start 150 mL were added and a sample of the homogenous light brown mixture was drawn. GC-Analyses of the sample showed complete splitting of the wax.

### Experiment 12:

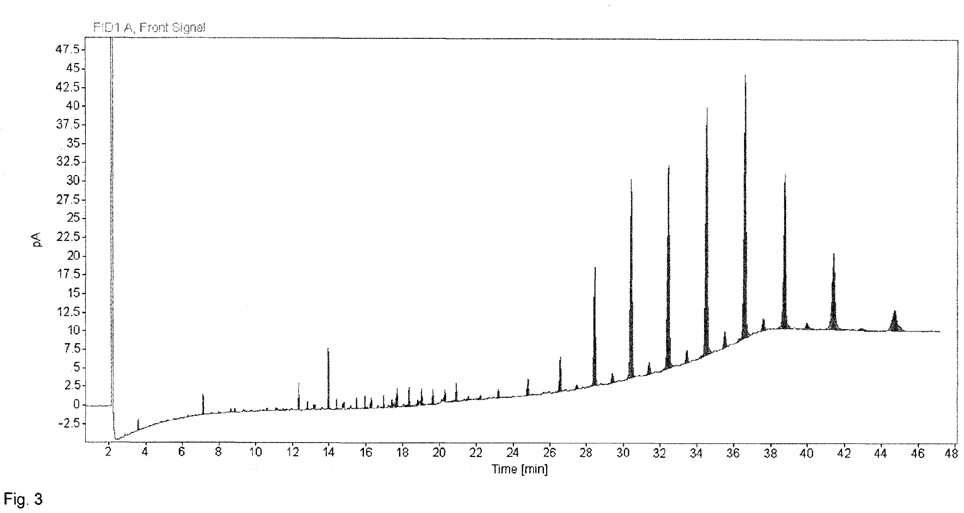

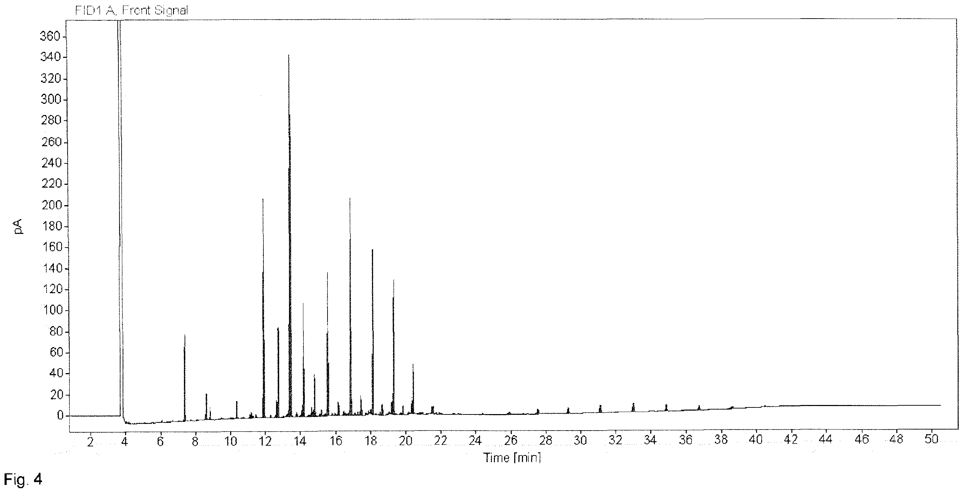

500 g RBW were liquefied at 100 °C. 56.0 g KOH (85%) were added with 32.7 mL H₂O under stirring. The oil bath was heated to 110 °C and the mixture stirred for 22 hours. The wax was completely split at this point and worked-up up with aqueous acid to gain a split wax with very low ester content.. Chromtogram 1 (Fig. 3) represents a chromatogram of raw RBW, Chromatogram 2 (Fig. 4) represents the chromatogram of the resulting split RBW.

### Experiment 13:

1.02 g C₁₆-O₂C₁₈ was added to a solution of 600 µL of a 29 wt.% KOH solution in water (1.57 eq.) at room temperature. The mixture was heated to 100 °C and stirred overnight for 17 hours, worked up and analysed by GC. The ester was hydrolysed to over 99.5 %.

### Oxidation of Alcohols

Procedure 3: 1.0 g of split RBW (gained by the previous experiment), the specified amounts of H₂SO₄ and of Cr(VI) as Na₂Cr₂O₇ were added into the reaction vessel, heated to 90 °C and stirred for 18 h.

**Table 3: Experiments according to Procedure 3.**

| **#** | **Substrate** | **H₂SO₄ (50 wt.%) [9]** | **Cr(VI) [eq.]** | **Ester ratio [%(I)]** | **X_{C30} [%]** |
|---|---|---|---|---|---|
| 14 | RBW (split) | 1.02 | 1.5 | 8.5 | 87.0 |
| 15 | RBW (split) | 3.40 | 5.0 | 13.0 | 87.0 |

### Combination of Ester Cleavage and Oxidation of Alcohols

### Experiment 16:

500.0 g RBW were heated in a 4 L three-neck flask equipped with an overhead-stirrer and reflux-condenser, suspended in an oil bath which was set to 115 °C. When the wax reached a temperature of app. 110 °C, 40.02 g NaOH dissolved in 20 mL hot water were added hot (app. 110 °C). At minute 15 (after NaOH addition) the oil bath was set to 120 °C. The mixture was allowed to react overnight (GC showed complete splitting), before 500 mL H₂O and 20 g HOAc (0.33 mol, 0.5eq.) in 1000 mL H₂O were added and the oil bath temperature was set to 95 °C. 500 g diluted H₂SO₄ (8 wt.%) were added slowly within 4 hours. Additional 5 g of H₂SO₄ were added with 800 mL H₂O before aqueous and organic phase separated. The wax was worked-up before the 2.52 g CA and 3.02 g OH-TEMPO were added with 920 mL H₂O. 12.4 g NaOCI (5 % free Cl₂) were added and the addition of 90 g NaClO₂ in 300 mL H₂O was started with a peristaltic pump within 2.5 hours. The mixture was allowed to stir overnight before heating and cooling was turned off. Cooled to room temperature the aqueous phase was decanted, the wax was heated to 90 °C, washed with water. GC showed a C₃₀ conversion of app. 90 % and the acid value was determined to be 114.

### Experiment 17:

20.02 g RBW were heated in a flask equipped with an overhead-stirrer in an oil bath which was set to 110 °C. When the wax was liquefied, 2.027 g NaOH dissolved in 5 mL H₂O were added within minute 0-6. A sample was drawn at minute 10, showing partial first splitting. 15 mL H₂O were added after minute 30 and a sample was taken at minute 90, showing complete splitting of all esters. 6.01 g HOAc were dissolved in 60 mL H₂O and slowly added, while simultaneously letting the oil bath cool to 100 °C (minute 170-180). pH value was 4-5 when 36.5 mg TEMPO were added in 3 mL HOAc, followed by 0.2 mL NaOCI (5 % free Cl₂). 4.502 g NaClO₂ were added in 20 mL H₂O from minute 200-220. pH value was determined to be 4-5. At minute 230 a sample was taken, which showed app. 60 % oxidation of C₃₀. Evolution of a greenish gas, supposedly Cl_{2(g)} was observed at minute 240. At minute 300 a sample was taken which showed app. 92 % oxidation. The aqueous phase was discarded and the organic phase was washed 3 times with 40 mL H₂O. The resulting product was dried under reduced pressure (10⁻¹ bar) at 95 °C. The acid value was determined to be 130.

### Experiment 18:

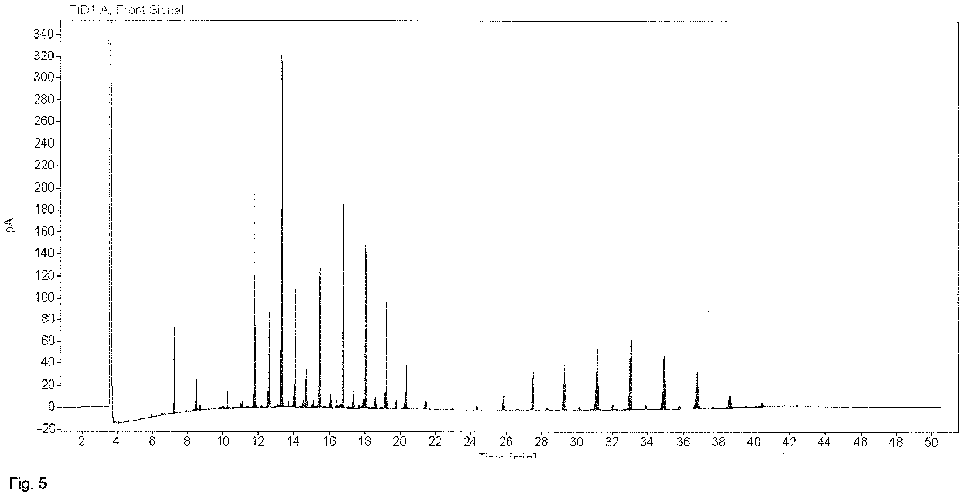

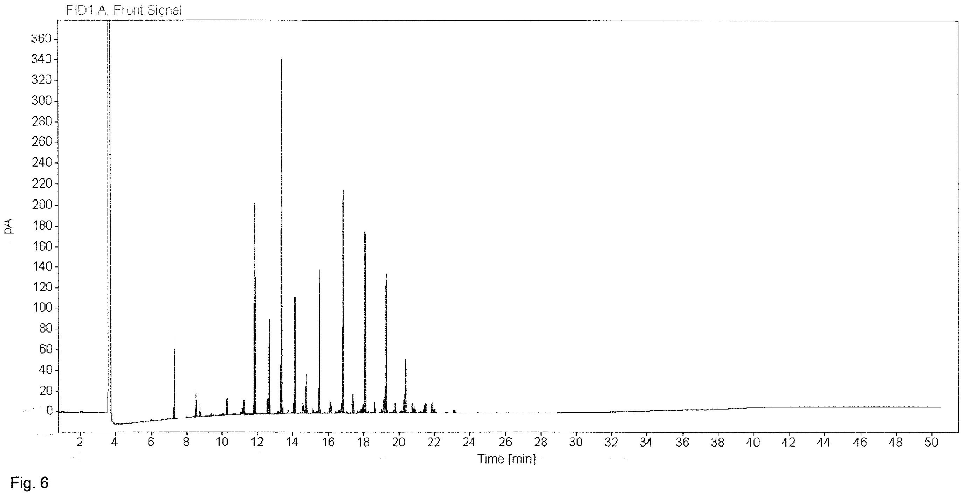

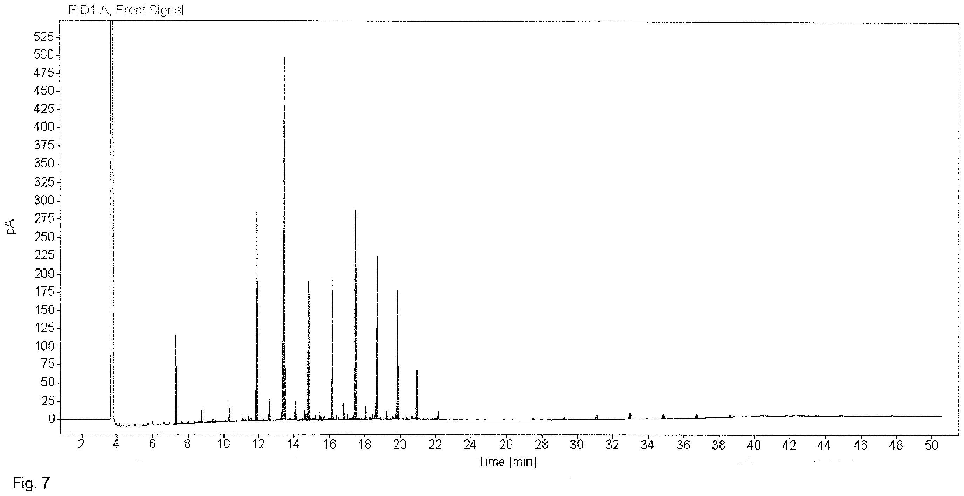

1 000 g RBW were heated in a 4 L three-neck flask equipped with an overhead-stirrer and reflux-condenser, suspended in an oil bath which was set to 120 °C. The reflux condenser was open at the top to prevent pressure built up. When the wax reached 110 °C, 112.1 g KOH (85 %, 1.7 mol, 1.28 eq.) dissolved in 70 mL H₂O were slowly added. 30 minutes after the addition over ¾ of the wax were already split, while 50 minutes after the addition the GC showed complete splitting of the esters. 500 mL H₂O were slowly added and the oil bath temperature set to 95 °C. Thereafter 132.2 g CA dissolved in 300 mL H₂O were added over 2 hours. The oil bath was set to 90 °C and 6.00 g OH-TEMPO were added in 120 mL H₂O. Successively 40 mL NaOCI (5 % free Cl₂) were added and the addition of 180 g NaClO₂ in 300 mL H₂O over 2.5 hours was started. 1.5 hours after completed addition a sample showed >94 % conversion of C₃₀. The product was thereafter worked-up and washed several times, before 1 031 g of oxidized RBW were gained, whose acid value was determined to be 132. Chromatogram 3 (Fig. 5) was obtained 30 minutes after addition of the lye. Chromatogram 4 (Fig. 6) was obtained 50 minutes after addition of the lye. Chromatogram 5 (Fig. 7) is a chromatogram of the final product after oxidation of the split wax.

## Claims

1. A process for the oxidation of an ester via an ester cleavage reaction followed by the oxidation of the alcohol component of the ester, said process comprising
a step of subjecting a starting material comprising one or more esters of a carboxylic acid having a carbon number of 14 or more with an alcohol having a carbon number of 14 or more to an ester cleavage reaction wherein at least a part of the one or more esters is hydrolyzed to obtain a mixture of one or more carboxylic acid salts with a carbon number of 14 or more and one or more alcohols with a carbon number of 14 or more, and wherein the ester cleavage reaction is carried out under atmospheric pressure and in a temperature range of 90 to 140 °C using an aqueous base with a concentration of the base in water of 20 wt.% or more;
a step of acidifying the one or more carboxylic acid salts with a carbon number of 14 or more to obtain one or more carboxylic acids; and
a step of oxidizing at least a part of the one or more alcohols with a carbon number of 14 or more to obtain one or more carboxylic acids;
wherein the step of acidifying the carboxylic acid salts and the step of oxidizing the alcohols can be carried out concurrently or successively.

2. The process of claim 1, wherein the starting material comprises one or more esters of a carboxylic acid with a carbon number of 16 or more and 42 or less, more preferably 20 or more and 40 or less and an alcohol with a carbon number of 16 or more and 42 or less, more preferably 20 or more and 40 or less.

3. The process of claim 1 or 2, wherein the starting material comprises one or more esters of the formula
CH₃-(CH₂)ₘ-C(O)-O-(CH₂)ₙ-CH₃,
wherein m+2 corresponds to the carbon number of the carboxylic acid component of the ester and n+1 corresponds to the carbon number of the alcohol component of the ester.

4. The process of any of claims 1 to 3, wherein the starting material is a wax selected from rice bran wax, sunflower wax, carnauba wax, candelilla wax, sugarcane wax and beeswax.

5. The process of any of claims 1 to 4, wherein the ester cleavage reaction is carried out at a temperature in the range of 90 to 140°C.

6. The process of any of claims 1 to 5, wherein the base is selected from NaOH and KOH.

7. The process of any of claims 1 to 6, wherein the concentration of the base in water is 25 wt% or more.

8. The process of any of claims 1 to 7, wherein the amount of the aqueous base used in the ester cleavage reaction is such that 1.0 molar equivalents of the base or more are available per mole of ester to be hydrolyzed in the ester cleavage reaction.

9. The process of any of claims 1 to 8, wherein the ester cleavage reaction is carried out in the absence of an organic solvent.

10. The process of any of claims 1 to 9, wherein the step of oxidizing at least a part of the one or more alcohols with a carbon number of 14 to obtain one or more carboxylic acids comprises the oxidation of the alcohols using an oxoammonium cation and a co-oxidant as oxidizing agents at a reaction temperature of 50°C or higher to obtain the carboxylic acids.

11. The process of claim 10, wherein the step of oxidizing at least a part of the one or more
alcohols with a carbon number of 14 which are provided by the ester cleavage reaction to obtain one or more carboxylic acids comprises
providing a reaction mixture which comprises (i) the one or more alcohols with a carbon number of 14 and (ii) a precursor compound for an oxoammonium cation, and (iii) a co-oxidant, allowing the precursor compound to form an oxoammonium cation, and
oxidizing the alcohols using the oxoammonium cation and the co-oxidant as oxidizing agents at a reaction temperature of 50°C or higher to obtain one or more carboxylic acids.

12. The process of claim 11, wherein the precursor compound is an aminoxyl radical selected from 2,2,6,6-tetramethylpiperidine-1-oxyl (TEMPO) and from derivatives thereof carrying a substituent at the 4-position of the piperidine ring, from 9-azabicyclo[3.3.1]nonane N-oxyl (ABNO), and from 2-azaadamantane N-oxyl (AZADO), 1-methyl-2-azaadamantane N-oxyl (1-Me-AZADO) and 1,3-dimethyl-2-azaadamantane N-oxyl (1,3-dimethyl-AZADO).

13. The process of any of claims 10 to 12, wherein the co-oxidant is a chlorite or a bromite.

14. The process of any of claims 10 to 13, wherein the step of oxidizing the alcohols is carried out in the absence of an organic solvent.

15. The process of any of claims 10 to 14, wherein the step of oxidizing the alcohols is carried out in the presence of an aqueous medium.

## Patentansprüche

1. Verfahren zur Oxidation eines Esters über eine Esterspaltungsreaktion, gefolgt von der Oxidation der Alkoholkomponente des Esters, wobei das Verfahren
einen Schritt, bei dem ein Ausgangsmaterial, das einen oder mehrere Ester einer Carbonsäure mit einer Kohlenstoffzahl von 14 oder mehr umfasst, mit einem Alkohol mit einer Kohlenstoffzahl von 14 oder mehr einer Esterspaltungsreaktion unterzogen wird, wobei mindestens ein Teil des einen oder der mehreren Ester hydrolysiert wird, um eine Mischung aus einem oder mehreren Carbonsäuresalzen mit einer Kohlenstoffzahl von 14 oder mehr und einem oder mehreren Alkoholen mit einer Kohlenstoffzahl von 14 oder mehr zu erhalten, und wobei die Esterspaltungsreaktion unter Atmosphärendruck und in einem Temperaturbereich von 90 bis 140 °C unter Verwendung einer wässrigen Base mit einer Konzentration der Base in Wasser von 20 Gew. % oder mehr durchgeführt wird;
einen Schritt des Ansäuerns des einen oder der mehreren Carbonsäuresalze mit einer Kohlenstoffzahl von 14 oder mehr, um eine oder mehrere Carbonsäuren zu erhalten; und
einen Schritt des Oxidierens mindestens eines Teils des einen oder der mehreren Alkohole mit einer Kohlenstoffzahl von 14 oder mehr, um eine oder mehrere Carbonsäuren zu erhalten, umfasst;
wobei der Schritt des Ansäuerns der Carbonsäuresalze und der Schritt des Oxidierens der Alkohole gleichzeitig oder nacheinander durchgeführt werden können.

2. Verfahren nach Anspruch 1, wobei das Ausgangsmaterial einen oder mehrere Ester einer Carbonsäure mit einer Kohlenstoffzahl von 16 oder mehr und 42 oder weniger, bevorzugter 20 oder mehr und 40 oder weniger, und einen Alkohol mit einer Kohlenstoffzahl von 16 oder mehr und 42 oder weniger, bevorzugter 20 oder mehr und 40 oder weniger, umfasst.

3. Verfahren nach Anspruch 1 oder 2, wobei das Ausgangsmaterial einen oder mehrere Ester der Formel
CH₃-(CH₂)ₘ-C(O)-O-(CH₂)ₙ-CH₃
umfasst, wobei m+2 der Kohlenstoffzahl der Carbonsäurekomponente des Esters entspricht und n+1 der Kohlenstoffzahl der Alkoholkomponente des Esters entspricht.

4. Verfahren nach einem der Ansprüche 1 bis 3, wobei das Ausgangsmaterial ein Wachs ist, das aus Reiskleiewachs, Sonnenblumenwachs, Carnaubawachs, Candelillawachs, Zuckerrohrwachs und Bienenwachs ausgewählt ist.

5. Verfahren nach einem der Ansprüche 1 bis 4, wobei die Esterspaltungsreaktion bei einer Temperatur im Bereich von 90 bis 140°C durchgeführt wird.

6. Verfahren nach einem der Ansprüche 1 bis 5, wobei die Base ausgewählt ist aus NaOH und KOH.

7. Verfahren nach einem der Ansprüche 1 bis 6, wobei die Konzentration der Base in Wasser 25 Gew.-% oder mehr beträgt.

8. Verfahren nach einem der Ansprüche 1 bis 7, wobei die Menge der wässrigen Base, die in der Esterspaltungsreaktion verwendet wird, so ist, dass 1,0 molare Äquivalente der Base oder mehr pro Mol Ester, der in der Esterspaltungsreaktion hydrolysiert werden soll, verfügbar sind.

9. Verfahren nach einem der Ansprüche 1 bis 8, wobei die Esterspaltungsreaktion in Abwesenheit eines organischen Lösungsmittels durchgeführt wird.

10. Verfahren nach einem der Ansprüche 1 bis 9, wobei der Schritt des Oxidierens mindestens eines Teils des einen oder der mehreren Alkohole mit einer Kohlenstoffzahl von 14, um eine oder mehrere Carbonsäuren zu erhalten, die Oxidation der Alkohole unter Verwendung eines Oxoammonium-Kations und eines Co-Oxidationsmittels als Oxidationsmittel bei einer Reaktionstemperatur von 50°C oder höher umfasst, um die Carbonsäuren zu erhalten.

11. Verfahren nach Anspruch 10, wobei der Schritt des Oxidierens mindestens eines Teils des einen oder der mehreren Alkohole mit einer Kohlenstoffzahl von 14, die durch die Esterspaltungsreaktion bereitgestellt werden, um eine oder mehrere Carbonsäuren zu erhalten,
das Bereitstellen eines Reaktionsgemischs, das (i) den einen oder die mehreren Alkohole mit einer Kohlenstoffzahl von 14 und (ii) eine Vorläuferverbindung für ein Oxoammoniumkation und (iii) ein Co-Oxidationsmittel umfasst,
das Ermöglichen, dass die Vorläuferverbindung ein Oxoammoniumkation bildet, und
das Oxidieren der Alkohole unter Verwendung des Oxoammoniumkations und des Co-Oxidationsmittels als Oxidationsmittel bei einer Reaktionstemperatur von 50°C oder höher, um eine oder mehrere Carbonsäuren zu erhalten, umfasst.

12. Verfahren nach Anspruch 11, wobei die Vorläuferverbindung ein Aminoxylradikal ist, ausgewählt aus 2,2,6,6-Tetramethylpiperidin-1-oxyl (TEMPO) und Derivaten davon, die einen Substituenten in der 4-Position des Piperidinrings tragen, aus 9-Azabicyclo[3.3.1]nonan-N-oxyl (ABNO), und aus 2-Azaadamantan-N-oxyl (AZADO), 1-Methyl-2-azaadamantan-N-oxyl (1-Me-AZADO) und 1,3-Dimethyl-2-azaadamantan-N-oxyl (1,3-Dimethyl-AZADO).

13. Verfahren nach einem der Ansprüche 10 bis 12, wobei das Co-Oxidationsmittel ein Chlorit oder ein Bromit ist.

14. Verfahren nach einem der Ansprüche 10 bis 13, wobei der Schritt des Oxidierens der Alkohole in Abwesenheit eines organischen Lösungsmittels durchgeführt wird.

15. Verfahren nach einem der Ansprüche 10 bis 14, wobei der Schritt des Oxidierens der Alkohole in Gegenwart eines wässrigen Mediums durchgeführt wird.

## Revendications

1. Procédé pour l'oxydation d'un ester par l'intermédiaire d'une réaction de clivage d'ester suivie de l'oxydation du composant alcool de l'ester, ledit procédé comprenant
une étape consistant à soumettre un produit de départ comprenant un ou plusieurs esters d'un acide carboxylique ayant un nombre d'atomes de carbone de 14 ou plus avec un alcool ayant un nombre d'atomes de carbone de 14 ou plus à une réaction de clivage d'ester, au moins une partie desdits un ou plusieurs esters étant hydrolysée pour obtenir un mélange d'un ou de plusieurs sels d'acides carboxyliques ayant un nombre d'atomes de carbone de 14 ou plus et d'un ou de plusieurs alcools ayant un nombre d'atomes de carbones de 14 ou plus, et la réaction de clivage d'ester étant effectuée sous la pression atmosphérique et dans une plage de température de 90 à 140 °C à l'aide d'une base aqueuse ayant une concentration de la base dans de l'eau de 20 % en poids ou plus ;
une étape consistant à acidifier lesdits un ou plusieurs sels d'acides carboxyliques ayant un nombre d'atomes de carbone de 14 ou plus pour obtenir un ou plusieurs acides carboxyliques ; et
une étape consistant à oxyder au moins une partie desdits un ou plusieurs alcools ayant un nombre d'atomes de carbone de 14 ou plus pour obtenir un ou plusieurs acides carboxyliques ;
l'étape consistant à acidifier les sels d'acides carboxyliques et l'étape consistant à oxyder les alcools pouvant être effectuées simultanément ou successivement.

2. Procédé selon la revendication 1, dans lequel le produit de départ comprend un ou plusieurs esters d'un acide carboxylique ayant un nombre d'atomes de carbone de 16 ou plus et 42 ou moins, plus préférablement 20 ou plus et 40 ou moins et d'un alcool ayant un nombre d'atomes de carbone de 16 ou plus et 42 ou moins, plus préférablement 20 ou plus et 40 ou moins.

3. Procédé selon la revendication 1 ou 2, dans lequel le produit de départ comprend un ou plusieurs esters de formule
CH₃-(CH₂)ₘ-C(O)-O-(CH₂)ₙ-CH₃,
où m+2 correspond au nombre d'atomes de carbone du composant acide carboxylique de l'ester et n+1 correspond au nombre d'atomes de carbone du composant alcool de l'ester.

4. Procédé selon l'une quelconque des revendications 1 à 3, dans lequel le produit de départ est une cire choisie parmi le cire de son de riz, la cire de tournesol, la cire de carnauba, la cire de candelilla, la cire de sucre de canne et la cire d'abeille.

5. Procédé selon l'une quelconque des revendications 1 à 4, dans lequel la réaction de clivage d'ester est effectuée à une température dans la plage de 90 à 140 °C.

6. Procédé selon l'une quelconque des revendications 1 à 5, dans lequel la base est choisie parmi NaOH et KOH.

7. Procédé selon l'une quelconque des revendications 1 à 6, dans lequel la concentration de la base dans de l'eau est de 25 % en poids ou plus.

8. Procédé selon l'une quelconque des revendications 1 à 7, dans lequel la quantité de la base aqueuse utilisée dans la réaction de clivage d'ester est telle que 1,0 équivalent molaire de la base ou plus est disponible par mole d'ester à hydrolyser dans la réaction de clivage d'ester.

9. Procédé selon l'une quelconque des revendications 1 à 8, dans lequel la réaction de clivage d'ester est effectuée en l'absence d'un solvant organique.

10. Procédé selon l'une quelconque des revendications 1 à 9, dans lequel l'étape consistant à oxyder au moins une partie desdits un ou plusieurs alcools ayant un nombre d'atomes de carbone de 14 pour obtenir un ou plusieurs acides carboxyliques comprend l'oxydation des alcools avec utilisation d'un cation oxoammonium et d'un co-oxydant en tant qu'agents d'oxydation à une température de réaction de 50°C ou plus pour obtenir les acides carboxyliques.

11. Procédé selon la revendication 10, dans lequel l'étape consistant à oxyder au moins une partie desdits un ou plusieurs alcools ayant un nombre d'atomes de carbone de 14 qui sont produits par la réaction de clivage d'ester pour obtenir un ou plusieurs acides carboxyliques comprend
fournir un mélange réactionnel qui comprend (i) lesdits un ou plusieurs alcools ayant un nombre d'atomes de carbone de 14 et (ii) un composé précurseur pour un cation oxoammonium, et (iii) un co-oxydant,
permettre au composé précurseur de former un cation oxoammonium, et
oxyder les alcools en utilisant le cation oxoammonium et le co-oxydant en tant qu'agents d'oxydation à une température de réaction de 50°C ou plus pour obtenir lesdits un ou plusieurs acides carboxyliques.

12. Procédé selon la revendication 11, dans lequel le composé précurseur est un radical aminoxyle choisi parmi le 2,2,6,6-tétraméthylpipéridine-1-oxyle (TEMPO) et ses dérivés portant un substituant à la position 4 du cycle pipéridine, parmi le 9-azabicyclo[3.3.1]nonane N-oxyle (ABNO), et parmi le 2-azaadamantane N-oxyle (AZADO), le 1-méthyl-2-azaadamantane N-oxyle (1-Me-AZADO) et le 1,3-diméthyl-2-azaadamantane N-oxyle (1,3-diméthyl-AZADO).

13. Procédé selon l'une quelconque des revendications 10 à 12, dans lequel le co-oxydant est un chlorite ou un bromite.

14. Procédé selon l'une quelconque des revendications 10 à 13, dans lequel l'étape consistant à oxyder les alcools est effectuée en l'absence d'un solvant organique.

15. Procédé selon l'une quelconque des revendications 10 à 14, dans lequel l'étape consistant à oxyder les alcools est effectuée en la présence d'un milieu aqueux.
